Europäisches Patentamt

European Patent Office

Office européen des brevets

(19)

(11) Publication number: **0 058 539**

**A1**

(12) # EUROPEAN PATENT APPLICATION

(21) Application number: **82300714.1**

(22) Date of filing: **12.02.82**

(51) Int. Cl.³: **C 12 Q 1/00**
**C 12 Q 1/42**

(30) Priority: **12.02.81 GB 8104395**
**21.03.81 GB 8108928**
**02.04.81 GB 8110360**

(43) Date of publication of application:
**25.08.82 Bulletin 82/34**

(84) Designated Contracting States:
**AT BE CH DE FR GB IT LI NL SE**

(71) Applicant: **Self, Colin Henry**
**46 Lensfield Road**
**Cambridge, CB2 1EG(GB)**

(72) Inventor: **Self, Colin Henry**
**46 Lensfield Road**
**Cambridge, CB2 1EG(GB)**

(74) Representative: **Cole, William Gwyn**
**The Shires 3 Cricketfield Lane**
**Bishops Stortford Hertfordshire(GB)**

(54) Enzymatic detection method and use.

(57) A method of detecting an enzyme of a substrate therefore or a coenzyme therefor which enzyme is capable of making available a nicotinamide coenzyme which method comprises contacting a source of said enzyme and optionally its substrate or its coenzyme to be detected with a progenitor for a nicotinamide coenzyme whereby a nicotinamide coenzyme is made available which nicotinamide coenzyme starts a cyclic chemical reaction which interconverts the nicotinamide coenzyme and its reduced form and produces a detectable change which is detected is described.

EP 0 058 539 A1

Croydon Printing Company Ltd

TITLE MODIFIED
see front page

## DETECTION METHOD AND USE

The present invention relates to a method for the detection of an enzyme or a substrate thereof or a cofactor thereof and to the use of such detection method in medicine.

Raised levels of enzymes such as phosphatases capable of producing a nicotinamide coenzyme have been used to diagnose pathological conditions such as diseases of the liver, bone, kidney and prostate. Existing methods of detection of these enzymes are somewhat limited in terms of their sensitivity since they generally employ reagents such as phenylphosphate or nitrophenylphosphate or the like. Generally such methods have to be monitored with spectrophotometers sensitive in the range of 280 to 420nm which can be a disadvantage. Sensitive methods are known for detecting enzymes by "sandwich Elisa" but such techniques can be slow or difficult because of time taken to form the "sandwich". Effective non-Elisa

-2-

methods are known (see for example B.K. Choe et.al., Clin. Chem., 26/13, 1854-1859 (2980) but these tend to lack the sensitivity of Elisa methods or involve troublesome radioisotopes. Clearly it wound be an advantage if a less complicated or more sensitive method was available. Such a method would be yet more desirable if it could be adapted to produce a readily detectable change especially one which could be followed continuously visually. Such a method (non-Elisa) has now been discovered.

Accordingly the present invention provides a method of detecting an enzyme or a substrate therefor or a coenzyme therefor which enzyme is capable of making available a nicotinamide coenzyme which method comprises contacting a source of said enzyme and optionally its substrate or its coenzyme to be detected with a progenitor for a nicotinamide coenzyme whereby a nicotinamide coenzyme is made available which nicotinamide coenzyme starts a cyclic chemical reaction which interconverts the nicotinamide coenzyme and its reduced form and produces a detectable change which is detected.

Favourably the method of this invention is adapted to the detection of an enzyme or its substrate but most preferably the method of this invention is adapted to the detection of an enzyme.

When used herein the term "nicotinamide coenzyme" means nicotinamide adenine dinucleotide (hereinafter NAD) or nicotinamide adenine dinculeotide phosphate (hereinafter NADP) or reduced nicotinamide adenine dinucleotide (hereinafter NADH) or reduced nicotinamide adenine dinucleotide phosphate (hereinafter NADPH) or their analogues which partake in the same cyclic reaction which interconverts the analogue and its reduced form. The most apt nicotinamide coenzymes herein are NAD or NADH and the preferred nicotinamide coenzyme herein is NAD. When used herein the term "making available" means liberating or producing. When used herein the term "liberating" means releasing from a previously closed system (for example when a vesicle is ruptured) or freeing from a surface. When used herein the term "progenitor" means a precursor or system from which a compound is liberated. Most suitably, this invention is concerned with systems in which "making available" means "producing" and "progenitor" means "precursor".

-4-

When used herein the term "starts" means that the presence of the nicotinamide coenzyme allows a cycle to occur which would not otherwise occur to significant extent.

Aptly the method of this invention is adapted to the detection of NAD-kinase or its substrate in which case the nicotinamide coenzyme produced is NADP. Aptly the method of this invention is adapted to the detection of NADase or its substrate or a phosphoatase in which case the nicotinamide coenzyme produced is NAD. The detection of NAD-kinase and NADase is useful in the investigation of metabolic states.

A clinically more important class of enzyme is phosphatase since phosphatases are frequently useful to monitor disease states. Thus more aptly the method of this invention is adapted to the detection of a phosphatase in which case the nicotinamide coenzyme produced is normally and preferably NAD. It is one of the surprising features of the method of this invention that it may be adapted to disparate types of enzymes such as NAD-kinase, NADase and phosphatase.

Schematically one suitable representation of the form of this invention adapted to the detection of NAD-kinase is thus:

-5-

NAD

ATP

ADP

NAD-kinase

reduced product

reducible reagent

NADP

Oxidizable reagent

oxidized product

NADPH

The skilled worker will appreciate that this form
of the invention can be used not only to detect
NAD-kinase but also to detect ATP which is the substrate
for NAD-kinase. Indeed the skilled worker will appreciate
that this form of the invention may similarly be used to
detect the presence of a material which produces ATP.

Schematically one suitable representation of the
form of this invention adapted to the detection of
NADase is thus:

Nicotinamide

NAD-dihydroxy-
acetone

NADase

NAD

reduced product

reducible reagent

Oxidizable reagent

Oxidized product

NADH

-6-

The skilled worker will appreciate that this form of the invention can be used not only to detect NADase but also to detect a substrate therefor such as NAD-dihydroxyacetone or nicotinamide.

Schematically one suitable representation of the preferred form of this invention adapted to the detection of phosphatase is thus:

$$
\begin{array}{c}
\text{NADP} \\
\downarrow \quad \text{phosphatase} \\
\text{NAD} \\
\text{reduced product} \quad\quad \text{oxidizable reagent} \\
\text{reducible reagent} \quad\quad \text{oxidized product} \\
\text{NADH}
\end{array}
$$

The skilled worker will appreciate that this form of the invention may be adapted to the detection of phosphatases such as acid or alkaline phosphatase. The skilled worker will also appreciate that this form of the invention can also be used to detect a substrate for the phosphatase such as NADP. However it is preferable that this invention is adapted to the detection of the phosphatase rather than a substrate therefor since the need for a reliable sensitive and easily operated method of detecting phosphatase is great.

The change detected as a result of carrying out the method of this invention may be the removal of a reagent consumed by the operation cycle or it may be the production of a product produced by operation of the cycle. Preferably the change detected as a result of carrying out the method of this invention is due to the production of a product produced by operation of the cycle. The change detected as a result of carrying out the method of this invention may either (a) relate to removal of a reagent directly consumed by operation of the cycle or it may be the production of a product directly produced by operation of the cycle or (b) alternatively the change may be indirectly caused by the intermediacy of other reactions. Preferably the change detected is due to the direct removal or more preferably to the direct production of material.

The reagents employed will be chosen to produce a detectable change which is most preferably a change visible to the eye, for example the production or removal of a colour. Preferably the reagents are chosen so that at least one visibly coloured product is formed.

It has been found that by employing a reagent which may be oxidized or reduced as a result of operation the the cycle a readily detectable change may be made to occur. Most aptly therefor a reagent is employed which on oxidation or reduction is converted to a product which is visually detectable.

Normally the conversion of the oxidisable reagent to the oxidized product is enzymatically catalysed. The enzyme employed is favourably a dehydrogenase.

A suitable oxidisable reagent for use in the NADP/NADPH cycle is isocitrate (for example as the sodium salt) which is converted to $\alpha$-ketoglutarate (the oxidised product) in the presence of NADP-specific isocitrate dihydrogenase.

A suitable reducible reagent for use with NADPH/NADP cycle is oxidised glutathione which in the presence of glutathione reductase produces reduced glutathione which may be detected by its reaction with 5,5'-Dithio-bis-(2-nitrobenzoic acid) known as DTNB.

A suitable oxidisable reagent for use in the NAD/NADH cycle is lactate (for example the sodium salt) which is converted to pyruvate (the oxidised product) in the presence of lactate dehydrogenase. A very favoured

-9-

oxidisable reagent for use in the NAD/NADH cycle is ethanol which is converted to acetaldehyde (the oxidised product) in the presence of alcohol dehydrogenase.

Other oxidizable reagents which may be oxidized by their appropriate dehydrogenase include glyoxalate, acetaldehyde, D-gycerate, L-malate, dihydroorortic acid, $\alpha$-glycerophosphate, triosephosphate, D-glucose, lipoamide, lipoate, glutathione, L-$\beta$-hydroxybutyryl CoA, uridinediphosphoglucuronic acid, hydroxysteroids such as 3-$\alpha$ or $\beta$-hydroxysteroids and 17-$\beta$ hydroxysteroids.

A desirable reducible reagent for use in the NADP/NADPH or NAD/NADH cycle is thiazolyl blue (also known as MTT or 3-(4,5-dimethylthiazol-2-yl)-2,5-diphenyltetrazolium bromide) which is converted to a reduced, coloured readily detectable product. Tetrazolium salts which produce a colour change on reduction by a NAD/NADH cycle are a favoured class of reducible agents. This reduction is enhanced by an electron transfer reagent such as phenazine ethosulphate (also known as PES) or phenazine methosulphate. A preferred electron transfer reagent is phenazine ethosulphate. The reduction product of the thiazolyl blue produced during the operation of the method of this invention can be detected by the eye or monitored at 570nm if desired in a conventional manner. Other apt tetrazolium salts such as INT or NBT may replace MTT.

-10-

The use of the above reagents in a favoured form
of this invention may be represented schematically thus:

NADP

reduced form of                    phosphatase
thiazolyl blue

electron transfer            NAD        oxidisable reagent
reagent                                 dehydrogenase

Thiazolyl blue              NADH        oxidised product

The skilled worker will realise that cycles of the
type envisaged herein will be carried out in aqueous
solution and most suitable under mild conditions; that is
at non-extreme temperatures such as $1^{\circ}$ to $40^{\circ}$C and more
generally $5^{\circ}$ to $38^{\circ}$C and at a pH of from 5 to 10 and
more susually 5.5 to 9.3.

Most favourably this invention is adapted to the
detection of human phophatases. For the detection of
acid phosphatase the initial pH of the medium is mildly
acid, for example pH 4-6, for example from 4.6 to 5.6
(for example produced by a citrate buffer).

NADP is added to the mildly acid medium (for example
the aqueous citrate buffer) and then the resulting
solution is mixed with the supposed acid phosphatase solution.  Magnesium salts such as magnesium chloride
may be advantageously included in this solution.  This
mixture is then allowed to incubate.  The incubation
period will depend on the amount of phosphatase
anticipated in the sample.  If the sample is anticipated
to contain a high level of phophatase this period can
be as short as one minute, whereas if low level of acid
phosphatase is anticipated this period can be extended
to several hours.  If it is not known how much acid
phosphatase, if any, is likely to be present this period
can be extended for several hours.  At the end of the
incubation period the pH of the medium is raised to a
mildly alkaline pH, for example about 7.5 to 9.5.  At
this point the reagents required for completing the cycle
are added, for example a solution containing the
oxidisable reagent (for example alcohol), the oxidation
catalyst for the oxidisable reagent (alcohol dehydrogenase),
the reducible reagent (for example thiazolyl blue) and
the reduction catalyst for the reducible reagent
(phenazine ethosulphate). (In an alternative form the
reagents for completing the cycle may be added earlier,
for example with the NADP). The material being monitored
(the reduction product of the thiazolyl blue) can then
be observed over a suitable period which can be short, for example less than 1 minute dealing with rich sources of enzyme, or can be protracted, for example 1 or more hours dealing with a dilute solution of enzyme. In a preferred form of this invention a visibly coloured product is formed, for example a dark colour is produced on reduction of thiazolyl blue by operation of a cycle of the type hereinbefore suggested.

For the detection of alkaline phosphatase the pH of the medium will be mildly alkaline, for example at about pH 7.5 to 10.5, for example 9.3, for example as maintained by a suitable buffer such as an ethanolamine buffer. The solution will favourably also contain magnesium chloride or the like magnesium salt. The solution is mixed with the NADP solution and then the resulting solution is mixed with the supposed alkaline phosphatase solution. This mixture is then allowed to incubate as described in the preceeding paragraph. No change in the pH is normally required at the end of the incubation period when detecting alkaline phosphatase. The reagents required for completing the cycle are then added and the system monitored as described in the previous paragraph. Alternatively all reagents may be added at the same time in order to form a one step process or if desired a two step process may be employed by adding one reagent after the others.

-13-

Aptly the human phosphatase detected by the method of this invention is acid phosphatase.  Acid phosphatase is a marker for prostatic disease such as cancer so that high detected levels in blood or urine may be used as a diagnostic test.

Aptly the human phosphatase detected by the method of this invention is alkaline phosphatase.  Alkaline phosphatase is a marker for Pagent's disease, cholestatic liver disease, renal adenocarcinoma so that high detected levels in blood or urine may be used as a diagnostic test.

From the foregoing it will be realised that in a favoured aspect this invention provides a method of detecting human phosphatase which method comprises contacting a source of said enzyme with NADP whereby NAD is produced which NAD starts a cyclic chemical reaction which interconverts NAD to NADH and which also produces a visually detectable change.

Most aptly the cyclic chemical reaction is one the operation of which causes a chemical compound to be formed which is visually detectable.  Favourably the cyclic chemical reaction is adapted to cause the oxidation

-14-

of at least one reagent and the reduction of at least one reagent and at least one of products of said oxidation and said reduction is coloured. The cyclic chemical reaction is favourably one in which thiazolyl blue is reduced thereby giving rise to a visibly detectable product. Other tetrazolium salts may also be employed.

The cyclic chemical reaction is favourably one in which the oxidisable reagent is ethanol the oxidation of which is catalysed by alcohol dehydrogenase. Other oxidisable reagents have been named hereinbefore.

A highly favoured aspect of the present invention provides a method of detecting a phosphatase capable of producing NAD or NADH from NADP or NADPH which method comprises contacting a source of said phosphatase with NADP or NADPH whereby NAD or NADH is produced to take part in a cyclic chemical reaction which interconverts NAD and NADH and produces a detectable change which is detected.

As previously indicated a preferred form of this aspect of the invention comprises the detection of a phosphatase capable of producing NAD from NADP which comprises contacting a source of said phosphatase with NADP whereby NAD is produced to take part in a cyclic chemical reaction which interconverts NAD to NADH and produces a detectable change which is detected.

A less favoured form of this aspect of the invention comprises the detection of a phosphatase capable of producing NADH from NADHP which comprises contacting a source of said phosphatase with NADPH whereby NADH is produced to take part in a cyclic chemical reaction which interconverts NADH and NAD and produces a detectable change which is detected. This form of the invention can be represented by the following reaction scheme:

$$
\begin{array}{c}
\text{NADPH} \\
\downarrow \quad \text{phosphatase} \\
\text{oxidised product} \quad \text{NADH} \quad \text{reducible reagent} \\
\text{oxidizable reagent} \quad \text{NAD} \quad \text{reduced product}
\end{array}
$$

The NAD or NADH produced may be produced in the presence of the reagents required to complete the cyclic chemical reaction or less preferably one or more of these other reagents may be added subsequently.

A colour change in such a cycle may be used by employing Medola blue (which may also be employed in other NAD/NADH cycles described hereinbefore).

-16-

As previously indicated raised levels of phosphatase occur in certain malignant states. Thus for example in advanced malignant disease a placental type of alkaline phosphatase (Regan isoenzyme) can be produce. In a further aspect therefore this invention provides a method of diagnosing advanced malignant disease which comprises assaying a biological fluid from the person to be diagnosed using an assay for alkaline phosphatase as hereinbefore described. Suitable biological fluids include blood, serum, urine, biopsy homogenates or the like of which serum and blood are most apt.

In a somewhat more general form, this invention provides a method of diagnosing disease which is diagnosable by detecting an enzyme capable of making available a nicotinamide coenzyme which method comprises carrying out a method of detecting an enzyme as hereinbefore defined which enzyme has been removed from a patient. Most suitably the diagnostic method is adapted to the detection of a human phosphatase especially human acid phosphatase and preferably human acid phosphatase produced by a prostatic cancer.

Most aptly the diagnosis is produced on a fluid such as blood or serum or in some cases even urine.

One of the advantages of this invention is that the sensitivity allows small samples to be employed thus reducing trauma to the patient.

Also incorporated herein by cross reference are the disclosures of my co-pending British Patent Application No. 8108929 entitled "Separation Techniques and Assay" in so far as it pertains to phosphatase, their detection and use of said detection. The techniques of said application are particularly useful when investigations of specific isoenzymes are undertaken or when it is desirable to avoid contaminating substances.

Thus this invention provides a method of detecting an enzyme in a liquid which enzyme is capable of making available a nicotinamide coenzyme which method comprises (i) contacting the liquid with a support to which is attached a substance to which the enzyme will bind specifically without eliminating its enzymatic properties so that the enzyme becomes associated with said support, (ii) separating the support with its associated enzyme from the liquid and (iii) using the support with its associated enzyme in a detection method as hereinbefore described which produces a detectable change.

-18-

Most aptly the enzyme employed is a phosphatase as hereinbefore indicated. Most aptly the nicotinamide coenzyme is NAD as hereinbefore indicated. Most aptly the detectable change is brought out by oxidation or reduction as hereinbefore indicated. Most aptly the liquid will be human blood, serum, urine, tissue homogenate or the like as hereinbefore indicated.

The support used in this invention may be in the form of a discrete surface such as that of a stick or dish or filter system or in the form of small particles such as a powder or in the form of gel materials. Aptly the support may be polystyrene,

nylon, cross linked dextrans (such as sephadex, sepharose and agarose), polyacrylamide, bromacetyl-cellulose or the like. Such materials are presently known for the immobilization of non-enzymatic biological materials such as proteins, for example antigens, nucleic acids and the like. The skilled worker will appreciate that such supports can bind a wide range of materials so that, in conventional manner, in order to prevent uptake of unwanted enzymes or the like from the liquid the support will be neutralised to prevent this, for example by treatment with innocuous protein after the substance to which the enzyme to be detected is bound to the support.

The substance to be bound to the support will normally and preferably be an antibody to the enzyme with the proviso that the antibody employed will not be one that eliminates the enzymatic properties of the enzyme to be detected. Very desirably the antibody will be a monoclonal antibody.

The binding of the antibody (or other substance) to the support may be physical or chemical binding. Physical binding includes adsorption onto the surface

of the support and chemical binding may be ionic or covalent. Sandwich systems are also envisaged. The antibody may be bound in any manner as long as it is still capable of specifically binding the enzyme in a manner which will not eliminate the enzyme activity.

Alternatively the substance to be bound to the support may be some other substance with specific affinity for the enzyme in question, such as a substrate, substrate-analogue or inhibitor substance for that enzyme. Such approaches are possible with enzymes having more than one active enzymic site per enzyme molecule for - if only one site is taken up in binding to the support through the substrate, substrate analogue, inhibitor - the other site may still be available for catalytic activity.

Generally sufficient binding of the enzyme to the support with its bound substance will take place within $2\frac{1}{2}$ hours, for example 10 seconds to 30 minutes depending on the reaction parameters, concentrations employed and degree of accuracy required. Sometimes the binding will take place within 30 seconds to 10 minutes and preferably from 1 to 5 minutes. Mild agitation may be employed to facilitate the binding.

At the end of the binding stage the liquid may be separated and the support with the enzyme bound thereto washed. Generally it is preferred that this washing is thorough in order to remove unwanted unbound material. Washing may be carried out with water, saline, buffer or the like of which phosphate buffered saline is favoured. Suitably the washing liquid may include a mild surfactant such as a Tween.

At this point the support with the enzyme bound thereto may be promptly used in an assay for the enzyme.

If the detection method of this invention is to be used under circumstances when only very low levels of phosphatase are anticipated then it may be advantageous to add in the first instance to the suspected phosphatase the NADP in the absence of at least one component required to complete the cycle, maintain the mixture for a period (for example 10 minutes to two hours) and then add the remaining component or components.

-22-

From an alternative viewpoint this invention provides a method of detecting a phosphatase which method comprises contacting a source of said phosphatase with an organic phosphate which is dephosphoralatable by the phosphatase to a material which starts a cyclic chemical reaction which produces a detectable change which is detected.

Most aptly the cyclic chemical reaction is one comprising an oxidation and a reduction reaction, for example one in which NAD and NADH are interconverted. The detectable change is aptly produced as hereinbefore described. Thus, for example, the cycle may also employ reagents such as alcohol dehydrogenase (20 - 40 μ, for example 30-35 μ), alcohol (3-1200mM, for example 10-1000mM), MTT (0.05-0.5mM, for example 0.08-0.2mM) PES (0.05-3mM, for example 0.1-2mM) and may be started by the production of NAD from NADP (0.05-0.5mM, for example 0.08-0.2mM). Cyclic reactions of this type may be stopped when desired (for example after a preset time or when an intense enough colour has been created) by acidification, for example to pH 3 to 4 by addition of an acid, for example 50- 100 μl of acetic acid.or an equivalent amount of a similar weak acid.

-23-

The following Examples illustrate the invention:

Example 1.

A solution of acid phosphatase (0.024ml of acid phosphatase type III, Sigma London Chemical Company, diluted 1,000 fold from the original 60 units per ml concentration) was added to citrate buffer (0.206ml of 20mM buffer at pH 5.65) contained in a 1ml spectrophotometer cuvette. To this was added a solution of NADP (0.010ml of a 10mM solution), the contents of the cuvette mixed and then incubated at $23^{O}C$ for 5 minutes. To this solution was added ethanolamine buffer (0.64ml, 0.14M at pH 9.3) followed by thiazolyl blue (0.010ml of 10mM solution) pehanzine ethosulphate (0.010ml of 40mM solution) alcohol dehydrogenase (0.050ml of enzyme solution substantially free of NAD, (enzyme supplied by Sigma London Chemical Company, number A3263) containing approx. 37 Units of enzyme activity), and ethanol (0.050ml of 0.1M solution). The subsequent reaction was followed continuously in a spectrophotometer at 570nm, showing a significant continuously progressive increase in optical density. The colour change from pale yellow darkening to black was clearly visible to the eye by 15 minutes.

The same amount of acid phosphatase failed to produce a visibly detectable colour change when an assay was carried out using the conventional assay based upon p-nitrophenylphosphate as described in "Methods in Enzymatic Analysis" Volume 1 pages 495-496, Ed Bergmeyer, published by Verlag Chemie & Academic Press 1974.

Example 2

Alkaline phosphatase obtained from the Sigma (London) Chemical Company (from Bovine (Calf) Intestine, Type VII catalogue number P 4502) was diluted in ten-fold dilutions to $1:10^6$ and these solutions assayed at a constant temperature (approximately $37^O$) in the following manner for alkaline phosphatase activity.

Into a spectrophotometer cuvette designed to hold 1.0ml samples was put

| | | |
|---|---|---|
| 10μl | 10mM | thiazolyl blue |
| 10μl | 40mM | phenazine ethosulphate |
| 10μl | 10mM | NADP |
| 50μl | 1:10 dilution of ADH[§] | |
| 100μl | 0.1M | ethanol |
| 0.81ml | 0.14M | ethanolamine-HCl buffer at pH 9.3 and containing 5mM $MgCl_2$ |

§ same enzyme as Example 1

-25-

The assay was then initiated by the addition of 10µl of the example to be assayed.

The contents of the cuvette were mixed and the reaction followed either visually or specrophotometrically at 570nm.

At all concentrations of alkaline phosphatase tested (even that diluted to $1:10^6$ fold) an obvious reaction was seen to take place which was readily detectable visually as the solution turned from pale yellow to black. The reaction was also seen to proceed faster with the more concentrated samples indicating the quantitative nature of the system.

Thus for example at a dilution $1:10^4$ the rate of optical density change observed at six minutes past initiation was 0.18 optical density units per minute; at $1:10^5$ the rate of change was 0.05 density units per minute; and at $1:10^6$ the rate of change was 0.02 density units per minute. At all dilutions the rate of increase in optical density increased with time. (Quantitative measurements were made at 570nm).

Example 3

The alkaline phosphatase used in this example
is as obtainable from the Sigma Chemical Co (London)
Ltd Type VII from bovine (calf) intestine catalogue
number P4502 (1981). Antibody is raised against this
material by any of the standard procedures.
For example 0.5mg of the alkaline phosphatase
suspension is taken and made to 1.0ml with distilled
water and shaken gently. One ml of Freund's complete
adjuvant is then added and the mixture homogenised
through a connector between two hypodermic syringes
back and forth until a stable water-in-oil emulsion
is produced. Approximately 1ml of the emulsion is
then injected intramuscularly into each hindquarter
of a rabbit. The injections are repeated two and four weeks later with the same amount of alkaline phosphatase but without the added Freund's adjuvant. Blood is then taken from the immunised animal two weeks after the last injection and the immune-serum taken off after clotting has taken place. An IgG fraction of the serum is then obtained by the standard procedure of DEAE Cellulose Chromatography set out in Methods in Immunology by D.H.Cambell, J.S.Garvey, N.E.Cremer and D.H.Sussdorf, 2nd Edition (1970) pages 193-197, published by W.A.Benjamin, Reading Massachusetts. An aliquot of the IgG fraction is then diluted to a protein concentration of 0.5mg/ml in 0.05M carbonate buffer at pH 9.6 and is used to coat the wells of polystyrene micro-haemagglutination trays (Type M29 AR Microtitre, Dynatech Laboratories) by the addition of 0.3ml of diluted antiserum being added to each well and left either for 6-8 hours at room temperature or 1 hour at 37°C. The solutions are then taken out of the wells by suction and replaced with 0.2% lactalbium and left overnight. The wells are emptied and then washed three times with phosphate buffered saline containing 0.05% Tween 20 (PBS Tween). The plates are now ready for use.

Starting with a 1:1000 dilution, ten-fold dilutions of the original source of alkaline phosphatase are made down to $1:10^7$ and 0.25ml of each placed separately into the coated wells. The solutions are left for 30 minutes at room temperature ($23^\circ$C) and then removed by aspiration. The wells are again washed three times with PBS-Tween. The wells are then assayed for their alkaline phosphatase activity.

To each well is added 210ul of 0.14$\underline{M}$ ethanolamine-HCl buffer at pH 9.3 and containing 5$\underline{mM}$ $MgCl_2$ followed by 10ul 10$\underline{mM}$ thiazolylblue ('Sigma London Chemical Co), 10ul of 40 $\underline{mM}$ phenazine ethosulphate (Sigma London Chemical Co), 5ul of alcohol dehydrogenase (contains 37 units) especially free from NAD (Sigma London Chemical Co - catalogue number A3263) and 5ul of 1$\underline{M}$ ethanol. Reactions are initiated by the addition of 10ul of a 10$\underline{mM}$ solution of NADP (Sigma London Chemical Co) to each well, the plate gently agitated and left at room temperature. The solutions in the wells to which a higher concentration of alkaline phosphatase was added show a change of colour from pale yellow to black much faster than those which have been exposed to a lower concentration, indicating that the method not only detects the presence of alkaline phosphatase in the solution added to the wells but is also capable of quantification.

In a simple alternative, suitable (e.g. polystyrene) 1.0ml plastic colourimeter cuvettes are coated with the IgG fraction of the antiserum as above. One ml samples of the alkaline phosphatase dilutions are then placed in individual cuvettes and incubated and washed as above. To each cuvette is then added 0.96ml of 0.14$\underline{M}$ ethanolamine-HCl buffer at pH 9.3 and containing 5m$\underline{M}$ MgCl$_2$ followed by 10ul 10m$\underline{M}$ thiazolyl blue, 10ul 40m$\underline{M}$ phenazine ethosulphate, 5ul alcohol dehydrogenase (37 units) and 5$\mu$l 1$\underline{M}$ ethanol. Reactions are initiated as before by the addition of 10ul 10m$\underline{M}$ NADP and the solutions mixed. The colour changes may then be monitored by eye or at 570nm in a colourimeter for more precise quantification.

The general method is adaptable to the detection of other alkaline phosphatases including those from human origin, however, in each case it is usually advisable to prime the wells with a specific antiserum raised against the particular alkaline phosphatase in question. Furthermore, different alkaline phosphatases from human origin (such as from liver and bone) may be separately determined in a mixture of different types by means of IgG fractions directed specifically against particular types used to charge specific wells.

Example 4.

The method of Example 1 was carried amended as follows. The acid phosphatase to be assayed was added to a citrate buffer colution (0.0206ml of 5mM citrate buffer at pH 4.7 made 2mM in $MgCl_2$). To this was added 10 µl of 0.1M PES, 10µl of 10mM MTT, 32U of alcohol dehydrogenase (Sigma A 3263) and 30µl of absolute ethanol. NADP (10µl of a 10mM solution) was then added and the solution mixed and incubated at $23^{\circ}C$ for ten minutes. After incubation 100µl of 0.5M Tris buffer at pH 8.8 was added and the mixture incubated for a further three minutes. At this point a dark blue colour resulted and the reaction was stopped by the addition of 50µl glacial acetic acid.

WHAT IS CLAIMED IS:

1. A method of detecting an enzyme or a substrate therefor or a coenzyme therefor which enzyme is capable of making available a nicotinamide coenzyme which method comprises contacting a source of said enzyme and optionally its substrate or its coenzyme to be detected with a progenitor for a nicotinamide coenzyme whereby a nicotinamide coenzyme is made available which nicotinamide coenzyme starts a cyclic chemical reaction which interconverts the nicotinamide coenzyme and its reduced form and produces a detectable change which is detected.

2. A method as claimed in claim 1 for detecting an enzyme wherein the nicotinamide coenzyme is NAD or NADH.

3. A method as claimed in claims 1 or 2 of detecting a phosphatase which method comprises contacting a source of the phosphatase with a precursor for NAD whereby NAD is made available and starts a cyclic chemical reaction which interconverts NAD and NADH and produces a detectable change which is detected.

4. A method as claimed in claim 3 wherein the precursor for NAD is NADP.

2

5.    A method as claimed in claim 1 of detecting human phosphatase which method comprises contacting a source of said enzyme with NADP whereby NAD is produced which NAD starts a cyclic chemical reaction which interconverts NAD and NADH and which also produces a visually detectable change.

6.    A method as claimed in any of claims 1 to 5 wherein the detectable change is a visually detectable change produced by the oxidation or reduction of an oxidizable or reducible substrate as a result of operation of the cycle.

7.    A method as claimed in claim 6 wherein the visually detectable change is caused by the reduction of MTT.

8.    A method as claimed in any of claims 1 to 7 which method further comprises (i) contacting the liquid with a support to which is attached a substance to which the enzyme will bind specifically without eliminating its enzymatic properties so that the enzyme becomes associated with said support, (ii) separating the support with its associated enzyme from the liquid and (iii) using the support with its associated enzyme in a method of claims 1 to 7.

9.   A method of diagnosing disease which method comprises carrying out a method as claimed in any of claims 1 to 8 on a source of enzyme removed from a patient.

10.   A method as claimed in claim 9 for diagnosing prostatic acid cancer wherein the enzyme to be detected is human acid phosphatase.

11.   A method of detecting a phosphatase which method comprises contacting a source of phosphatase with an organic phosphate which is dephosphoralatable by the phosphatase to a material which starts a cyclic chemical reaction which produces a detectable change which is detected.

| Category | Citation of document with indication, where appropriate, of relevant passages | Relevant to claim |
|---|---|---|
| A,P | EP - A2 - 0 025 227 (MILES LABORATORIES INC.)<br>* claim 1 *<br>-- | 1 |
| A,P | Patent Abstracts of Japan<br>Vol. 5, No. 114, 23 July 1981<br>page 786C64<br>& JP - A - 56 - 51998<br>-- | 3 |
| A | DE - A - 1 959 410 (BOEHRINGER MANNHEIM GMBH)<br>* pages 2 to 4 *<br>-- | 5,6 |
| A | DE - A1 - 2 514 638 (INSTITUT FRESENIUS CHEMISCHE UND BIOLOGISCHE LABORATORIEN GMBH)<br>* claims 1 to 7 *<br>-- | 8 |
| A | CLINICAL CHEMISTRY, Vol. 26, No. 11, 1980, Easton, USA<br>P. VIHKO et al. "Rapid Radioimmunoassay for Prostate-Specific Acid Phosphatase in Human Serum"<br>pages 1544 to 1547<br>* abstract *<br>-- | 10 |
| A | US - A - 4 141 792 (H. HAYASHI et al.)<br>---- | |

**DOCUMENTS CONSIDERED TO BE RELEVANT**

**CLASSIFICATION OF THE APPLICATION (Int. Cl. 3)**

C 12 Q 1/00
C 12 Q 1/42

**TECHNICAL FIELDS SEARCHED (Int.Cl. 3)**

C 12 Q 1/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| Berlin | 28-04-1982 | SCHWARTZ |

EPO Form 1503.1 06.78